Europäisches Patentamt

(19) European Patent Office

Office européen des brevets

(11) Veröffentlichungsnummer: **0 005 742**
**B2**

(12) **NEUE EUROPÄISCHE PATENTSCHRIFT**

(45) Veröffentlichungstag der neuen Patentschrift:
17.04.85

(51) Int. Cl.⁴: **A 61 F 13/20**

(21) Anmeldenummer: 79101378.2

(22) Anmeldetag: 05.05.79

(54) Tupfer für medizinische, hygienische, kosmetische und ähnliche Zwecke.

(30) Priorität: 29.05.78 DE 2823332

(43) Veröffentlichungstag der Anmeldung:
12.12.79 Patentblatt 79/25

(45) Bekanntmachung des Hinweises auf die Patenterteilung:
24.03.82 Patentblatt 82/12

(45) Bekanntmachung des Hinweises auf die Entscheidung
über den Einspruch:
17.04.85 Patentblatt 85/16

(84) Benannte Vertragsstaaten:
AT BE CH FR GB IT LU NL SE

(56) Entgegenhaltungen:
DE - A - 2 552 172
US - A - 2 740 405
US - A - 2 755 805
US - A - 2 972 350
US - A - 3 089 495

(73) Patentinhaber: Rath, Ewald, Geranienstrasse 5,
D-5600 Wuppertal 21 (DE)

(72) Erfinder: Rath, Ewald, Geranienstrasse 5,
D-5600 Wuppertal 21 (DE)

(74) Vertreter: Peerbooms, Rudolf, Dipl.-Phys.,
Postfach 200 208 Dickmannstrasse 45C,
D-5600 Wuppertal 2 (DE)

EP 0 005 742 B2

BUNDESDRUCKEREI BERLIN

**Beschreibung**

Die Erfindung betrifft einen Tupfer für medizinische, hygienische, kosmetische und ähnliche Zwecke, bestehend aus einem mehrlagigen zugeschnittenen Gewebematerial, insbesondere Mullgewebematerial, welches zu einem Beutel mit nach innen eingestülptem, durch einen Gummiring eingefaßten Rand eingeschlagen ist, wobei die Schnittränder des Gewebematerials innerhalb der Beutelhülle liegen und die Beutelhülle allseits zwei- oder mehrlagig ist.

Tupfer oder Tampons aus Mullgewebe oder Gaze gelangen in erster Line zur Anwendung bei operativen Eingriffen an Mensch und Tier. Da sie zum Freimachen und Säubern von Wunden und Operationsstellen von Blut und Sekreten dienen, müssen sie saugfähig sein, dürfen sie sich nicht auflösen und dürfen sie keine Fäden oder Flusen in der Wunde hinterlassen. Andererseits sollen sie locker und weich anschmiegsam sein, damit Wunden sehr schonend behandelt werden können.

Es ist seit langem bekannt, Tupfer aus Mullgewebe zu verwenden, die vom Krankenhauspersonal in Heimarbeit hergestellt werden. Hierbei wird von quadratischen Mullgewebezuschnitten ausgegangen, die durch geschickte Faltungen und Verschlingungen zu Bällchen geformt werden, bei denen die Flusen abgebenden Gewebe-Schnittränder vollständig im Inneren liegen. Eine solche Herstellung ist jedoch sehr lohnkostenaufwendig und auch aus hygienischen Gründen unbefriedigend. Durch die DE-A-2 552 172 sind ferner manuell herzustellende Tupfer in zylindrischer Form bekannt, die aus einem in mehreren Lagen übereinander gewickeltem textilem Band bestehen, wobei die Stirnenden des Bandwickels nach innen gestopft sind, so daß die Schnittränder des Bandes im Tupferinneren liegen. Solche Tupfer müssen aber zur Erzielung eines sicheren Zusammenhaltes verhältnismäßig fest und stramm gestopft werden und weisen eine inhomogene Materialdichte auf.

Durch die US-A-2 972 350 ist ferner ein Tupfer bekannt, der aus zwei aufeinanderliegenden Bändern gewickelt ist, wobei der zylindrische Wickel um seine Mitte geknickt und die beiden Stirnenden des Wickels durch einen Bindefaden unter Bildung eines säckchenförmigen Tupfers zusammengefaßt sind. Auch ein solcher Tupfer ist sehr inhomogen in der Materialdichte und Saugfähigkeit und darüber hinaus äußerst aufwendig in der Herstellung.

Durch die US-A-2 740 405, Fig. 3, ist ferner ein sehr preiswert herstellbarer Tupfer bekannt, der aus einem einzigen Gazezuschnitt gefertigt ist, dessen Schnittränder ringsum durch einen Gummiring unter Bildung eines beutelförmigen, kugeligen Tupfers hindurch gesteckt sind. Bei diesem Tupfer stechen jedoch Fadenenden durch die Beutelhülle hindurch, bei denen es sich um die Enden von losen Fadenflusen oder auch um noch eingebundene Fäden handeln kann, was optisch nicht unterschieden werden kann. Auch wenn die losen Flusen abgesaugt sein sollten, bleibt der Arzt ständig noch durch vorstehende Fadenenden irritiert und verunsichert, weshalb dieser bekannte Tupfer in der Praxis keinen Anklang gefunden hat. Ferner sind Tupfer hergestellt worden, die aus einem einzigen Gazezuschnitt bestehen, der zunächst doppellagig gefaltet und anschließend mit seinen gefalteten Rändern durch einen Gummiring hindurchgestülpt ist.

Der Erfindung liegt die Aufgabe zugrunde, einen Tupfer der letztbeschriebenen Art zu schaffen, der bei der maschinellen Fertigung zuverlässig ohne Flusen oder abstehende Fadenenden anfällt.

Zur Lösung dieser Aufgabe ist erfindungsgemäß vorgesehen, daß der Tupfer aus zwei einzelnen, längsmittig aufeinanderliegenden, etwa oder exakt gleich langen, rechteckigen Zuschnitten besteht, von denen der innenliegende Zuschnitt eine Breite von etwa zweifachem Tupferdurchmesser besitzt und der außenliegende Zuschnitt mindestens doppelt so breit wie der innenliegende Zuschnitt ist.

Beim Einstülpen solcher Tupfer werden die an den Rändern der einzustülpenden Zuschnitte freiliegenden Fadenenden nicht mehr durch das zwei- oder mehrlagige Hüllengewebe hindurchgestoßen, sondern auch bei umfangreichster Massenproduktion zeigt sich, daß zwei- oder mehrlagige Hüllen alle Fadenenden und Flusen sicher auffangen und im Inneren des Tupfers zurückhalten. Dabei fallen insgesamt sehr homogene, weiche Tupfer an.

In Ausgestaltung der Erfindung kann vorgesehen werden, daß die beiden Zuschnitte eine solche Größe besitzen, daß sie nebeneinandergelegt ein Quadrat bilden. Bei einer solchen Ausführungsform, bei der der Tupfer aus zwei ungleich großen Rechteckzuschnitten gefertigt ist, ergeben sich eiförmige Tupfer.

Die Erfindung wird im folgenden anhand eines in der Zeichnung dargestellten Ausführungsbeispiels näher beschrieben. In der Zeichnung zeigt

Fig. 1 einen Tupfer nach der Erfindung in perspektivischer Darstellung,

Fig. 2 in verkleinertem Maßstab zwei ausgebreitete Mullgewebezuschnitte gemäß einer ersten Ausführungsform und

Fig. 3 einen Schnitt durch einen aus den Zuschnitten nach Fig. 2 gefertigten Tupfer.

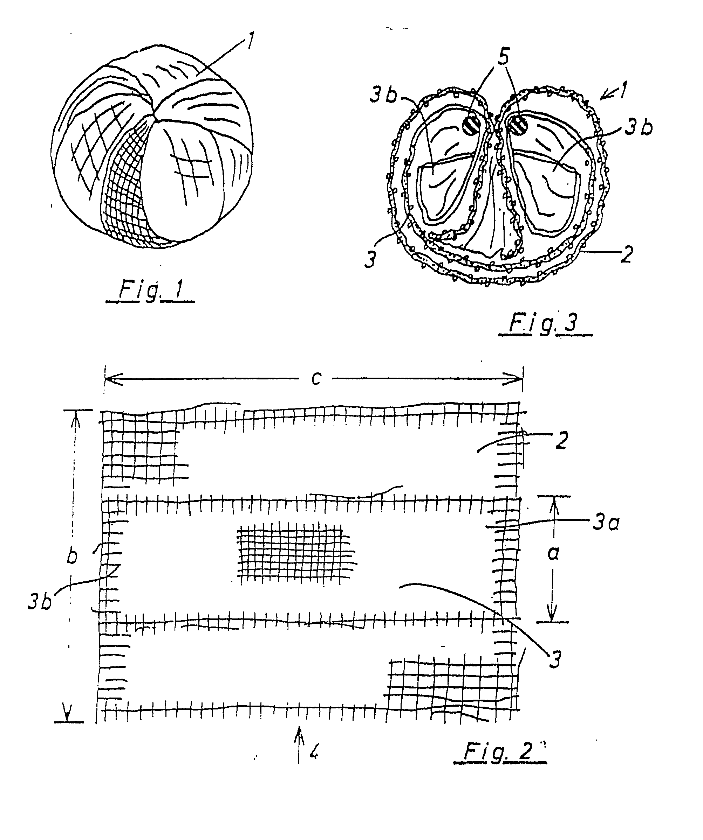

Fig. 1 zeigt einen zu einem Bällchen eingeschlagenen Tupfer 1, der aus zwei Mullgewebezuschnitten 2, 3 besteht. Die Zuschnitte 2, 3 sind längliche Rechtecke von gleicher Länge c. Der im fertigen Tupfer innenliegende Zuschnitt 3 hat eine Breite a, die etwa dem zweifachen Durchmesser des Tupfers 1 entspricht. Der außenliegende Zuschnitt 2 weist eine Breite b auf die etwa das Zweieinhalbfache der Breite a des Zuschnittes 3 beträgt. Werden die Zuschnitte 2, 3 nebeneinandergelegt, bilden sie ein Quadrat mit der Seitenlänge c. Der schmale Zuschnitt 3 liegt auf dem breiten Zuschnitt 2 auf, wobei sich ihre Längsmitten decken.

Fig. 3 zeigt einen Schnitt durch den maschinell aus den Zuschnitten 2, 3 nach Fig. 2 gefertigten Tupfer 1, wobei der Schnitt längs der Pfeilrichtung 4 (Fig. 2), also senkrecht zur Längsmitte der Zuschnitte 2, 3 geführt ist. Der innenliegende Gewebezuschnitt 3 liegt an etwas mehr als der Hälfte der Umfangsfläche des außenliegenden Zuschnittes 2 an. Die vier Ränder des Zuschnittes 2 und die schmalen Ränder mit angrenzenden Eckbereichen 3a, 3b des Zuschnittes 3 sind durch einen Gummiring 5 in den von den Zuschnittsmitten geformten Beutel gestülpt. Die Ränder der Zuschnitte 2, 3 treffen in dem Bereich des Beutels an Wandungsbereiche, in denen durch die aufeinanderliegenden Zuschnitte 2,3 eine Verdichtung vorgesehen ist. Abstehende Fadenenden werden dadurch daran gehindert, durch das Gewebe zu stoßen und aus dem Tupfer hervorzutreten. Durch die Wahl der Zuschnittsabmessungen wird ein Tupfer geschaffen, der in seinem ganzen Volumen etwa homogen ist und unabhängig von der Tupferstelle ein gleich gutes Saugverhalten aufweist.

**Patentansprüche**

1. Tupfer (1) für medizinische, hygienische, kosmetische und ähnliche Zwecke, bestehend aus einem mehrlagigen zugeschnittenen Gewebematerial (2, 3, 6), insbesondere Mullgewebematerial, welches zu einem Beutel mit nach innen eingestülptem, durch einen Gummiring (5, 7) eingefaßten Rand eingeschlagen ist, wobei die Schnittränder des Gewebematerials (2, 3, 6) innerhalb der Beutelhülle liegen, und die Beutelhülle allseits zwei- oder mehrlagig ist, dadurch gekennzeichnet, daß der Tupfer (1) aus zwei einzelnen längsmittig aufeinanderliegenden, etwa oder exakt gleich langen, rechteckigen Zuschnitten (2, 3) besteht, von denen der innenliegende Zuschnitt (3) eine Breite von etwa zweifachem Tupferdurchmesser besitzt, und der außenliegende Zuschnitt (2) mindestens doppelt so breit wie der innenliegende Zuschnitt (3) ist.

2. Tupfer nach Anspruch 1, dadurch gekennzeichnet, daß die beiden Zuschnitte (2, 3) eine solche Größe besitzen, daß sie nebeneinandergelegt ein Quadrat bilden.

**Claims**

1. A swab (1) for medical, hygienic, cosmetic and similar purposes, consisting of a cut-out fabric material (2, 3) with several layers, particularly a cut-out material of muslin cloth, which is tucked in to form a bag with an in-turned edge bound by a rubber ring (5), the cut edges of the fabric material (2, 3) lying inside the envelope of the bag and the envelope of the bag having two or more layers on all sides, characterised in that the swab (1) consists of two rectangular cut-out pieces (2, 3) which are substantially or precisely equal in length which lie concentrically lengthwise one upon the other, and of which the inner cut-out piece (3) has a width of about twice the diameter of the swab and the outer cut-out piece (2) is at least twice as wide as the inner cut-out piece (3).

2. A swab as claimed in Claim 1, characterised in that the two cut-out pieces (2, 3) have such a size that they form a square when laid side by side.

**Revendications**

1. Tampon (1) pour usage médical, hygiénique, cosmétique et analogue, constitué par du tissu découpé à plusieurs couches (2, 3), notamment du tissu de gaze, qui est replié pour former un sachet avec le bord rentré à l'intérieur et serré par un anneau élastique (5), les bords de coupe de tissu (2, 3) se trouvant à l'intérieur de l'enveloppe du sachet et l'enveloppe du sachet comportant de tous côtés deux ou plusieurs couches, caractérisé par le fait que le tampon (1) est constitué par deux morceaux (2, 3) séparés de coupes rectangulaires et de longueurs approximativement ou exactement égales, placés concentriquement l'un sur l'autre dans le sens longitudinal, le morceau intérieur (3) ayant une largeur environ double du diamètre du tampon et que le morceau extérieur (2) a une largeur au moins double de celle du morceau intérieur (3).

2. Tampon selon la revendication 1, caractérisé par le fait que les deux morceaux (2, 3) ont des dimensions telles que placés l'un à côté de l'autre ils forment un carré.

_Fig. 1_

_Fig. 3_

_Fig. 2_

5